# EUROPEAN PATENT SPECIFICATION

(11) **EP 1 604 209 B1**
(45) Date of publication and mention of the grant of the patent: **22.10.2008**
(21) Application number: 04719437.8
(22) Date of filing: 11.03.2004
(51) Int. Cl.: G01N 33/68, C12Q 1/37

(54) **SCREENING ASSAY**
SCREENING TEST
CRIBLAGE

(30) Priority: 12.03.2003 GB 0305656
(43) Date of publication of application: 14.12.2005
(73) Proprietor: BioInvent International AB, 223 70 Lund (SE)
(72) Inventor: NILSSON, Fredrik, S-216 19 Malmö (SE)
(74) Representative: Wainwright, Jane Helen
(86) International application number: PCT/EP2004/002566
(87) International publication number: WO 2004/081575

(56) References cited:
- WO-A-02/25287
- WO-A-02/060377
- WO-A-02/086081
- STOLL D ET AL: "PROTEIN MICROARRAY TECHNOLOGY" FRONTIERS IN BIOSCIENCE, XX, XX, vol. 7, 1 January 2002 (2002-01-01), pages C13-C32, XP008016809

## Description

The present invention relates to a screening assay.

### Background of the invention

Functional genomics is a research field with the aim of understanding what each gene does, how it is regulated and how different genes and gene products interact. An important aspect of functional genomics is to understand the structure and function of gene products, such as proteins, as well as being able to determine where, when and to what extent the genes are expressed. The term expression profiling or expression analysis usually encompasses both studies of mRNA expression (transcription analysis) and protein analysis (proteome analysis or proteomics).

Transcription analysis is typically performed using DNA on a micro-array format, allowing for parallel detection of thousands or tens of thousands of mRNA molecules simultaneously (e.g. using commercially available microarrays from e.g. Affymetrix, USA). Typically, these arrays are used to map distribution of transcripts in different tissues or to study differences in mRNA expression levels between e.g. healthy and sick individuals. Applications in drug development and drug discovery include target identification and patient stratification.

Protein expression profiling, or proteomics, is the global analysis of protein content in, for example, a tissue or cell population.

Two-dimensional electrophoresis coupled to mass spectrometry is a well-established technique for analysis of complex protein samples with sufficiently high resolving power to separate thousands of proteins. The major drawbacks of this technique are the lack of dynamic range (structural proteins and abundant metabolic enzymes tend to mask less abundant species), low throughput and high labour-intensity.

Surface Enhanced Laser Desorption/Ionisation (SELDI) (Weinberger et al, 2002, Journal of Chromatography B, 782, 307-316) is a technique based on the selective enrichment of a sub-population of proteins on an affinity surface (e.g. ion-exchange, reverse phase, antibodies) followed by mass spectrometric analysis by Matrix-Assisted Laser Desorption/Ionisation Time of Flight mass spectrometry (MALDI-TOF), a technique in which a co-precipitate of an UV-light absorbing matrix and biomolecules is irradiated by a nanosecond laser pulse. Most of the laser energy is absorbed by the matrix, which prevents unwanted fragmentation of the biomolecule. The ionised biomolecules are accelerated in an electrical field and separated according to their mass to charge ratio in a flight tube. However, the resolving power of this system is limited due to the restricted resolution of MALDI-TOF mass spectrometry for analysing large proteins and the sub-optimal separation of proteins achieved by the step-wise, solid-phase extraction type of separation technique employed.

Another alternative technique is referred to as isotope-coded affinity tags (ICAT) (Gygi et al, 1999, Nature Biotechnology, 17(1), 994-9), which utilises a cysteine-specific biotin tag to compare the protein expression pattern in two different samples. The tag allows for the extraction of cysteine-containing peptide from trypsin-digested protein mixtures, which reduces the complexity of the peptide fragments to level where analysis can be performed more easily. By using two different tags with different isotopic compositions, peptides originating from two different samples can be distinguished when analysed by mass spectrometry and a relative estimation of abundance can be obtained. However, the limitations of the ICAT technique include the insufficient reduction of complexity of highly complex samples, thus requiring further separation by liquid chromatography, and the fact that proteins lacking cysteine are not detected.

All of the above-mentioned techniques suffer from a set of limitations concerning, for example, sensitivity, speed, resolution and the ability to be applied to different types of proteins e.g. soluble and membrane bound proteins.

WO 02/086081 relates to a method for identifying a protein including cleaving the protein with a proteolytic agent to produce peptide fragments, contacting the fragments to an array comprising binding reagents, detecting binding and comparing binding to a reference set.

Other methods of analysing protein samples known in the prior art include the capture of trypsin-generated peptides using antibodies, each of which specifically binds a known peptide from a known protein (Scrivener, E. et al., 2003, Proteomics 3(2), 122-8; WO 02/25287). The captured peptides are then characterised by MALDI-TOF mass spectrometry. A similar approach is described by Nelson et al (1995, Anal Chem 67, 1153-8) where specific antibodies capture intact proteins and the captured proteins are eluted and analyses by mass spectrometry.

Both these,approaches presuppose the identity of the protein components to be analysed and require generation of binding molecules for each individual protein. Thus, to design an array to detect and measure e.g. 2000 proteins, these 2000 proteins or peptides must be isolated or synthesised followed by generation of 2000 specific antibodies or other binding molecules. In contrast, the present invention may detect a large number of peptides, such as 10,000, which may represent as many proteins, by using far fewer, such as only 200, different binders.

### Description of the invention

Accordingly, a first aspect of the present invention provides a method for analysing a heterogeneous sample of peptides, or protein or peptide fragments, the method comprising-
(a) separating the heterogeneous sample of peptides, or protein or peptide fragments, into heterogeneous classes by binding members of each class to a spaced apart defined location on an array, wherein members of each class have a motif common to that class; and
(b) characterising the peptides or protein or peptide fragments, in each class by determining the mass of peptides, or protein or peptide fragments, in heterogeneous classes, and determining the abundance of peptides, or protein or peptide fragments, of different mass in the heterogeneous classes.

The heterogeneous sample of peptides or proteins may be extracted from a cell or tissue sample, or derived from fragmentation of a heterogeneous sample of peptides and proteins extracted form a cell or tissue sample, typically (but not necessarily) of human origin. The cell or tissue sample may be derived from normal or diseased tissue. The cell or tissue sample may be derived from tissues at various states of differentiation or activity. Additional appropriate sources of proteins and peptides includes prokaryotes, eukaryotic cell lines, tissue materials from knockout mice and other animal models as well as transgenic plants and plant material.

The heterogeneous sample may be processed before analysis to remove particularly abundant proteins or peptides, such as albumin and/or immunoglobulins in a serum sample, or to enrich a sample for a particular protein or peptide or group of proteins or peptides.

Each heterogeneous class of peptides or proteins consists of all peptides or proteins in the heterogeneous sample that will bind to a specific binding molecule present on the array. The binding molecule is selected for its ability to bind a motif, rather than a particular protein or peptide, and so a binding molecule can bind different types of proteins and peptides containing the same motif. Preferably each binding molecule is specific for a given motif. Thus, a heterogeneous class of proteins and peptides bound by a given binding molecule in a method of the present invention typically comprises, as a mean average, at least two, more typically greater than two, such as 10, 20, 50, 100, 200, 500, 1000 or more, different types of protein or peptide. By "different type" we include the meaning of proteins and peptides differing in amino acid sequence, mass, post-translational modification and the like.

Accordingly, proteins and peptides are classified by the present invention based on their ability to be captured and retained by a specific binding molecule. A heterogeneous class of peptides or proteins will bind to specific binding molecule due to the presence of a motif common to all members of a particular class. The identity of the motif bound in each class of peptides is, therefore, a consequence of the binding specificity of the binding molecule that defines that class.

The motif may be a linear or non-linear sequence of amino acids such as four, five, six, seven, eight, nine, ten or more amino acids. A linear motif is formed from contiguous amino acids. A non-linear motif comprises amino acids that are non-adjacent in the sequence but are brought in close proximity to each other as a result of the three-dimensional folding of the protein or peptide.

Binding molecules on the array may be specific to sequences at particular locations within a protein or peptide, such as sequences at the C-terminus, the N-terminus, or at a defined position relative to an internal feature, such as a sequence or a modified amino acid. For example, all binding molecules on the array may be specific for C-terminal sequences, but each type of binding molecule may be specific for a different C-terminal sequence than other types of binding molecule on the array.

Similarly, the binding molecules on the array may be specific to sequences that contain a mixture of 'constant' and variable amino acids. The constant amino acids (as defined further below) can provide a constant feature common to all motifs bound by all binding molecules on the array. However, the exact identity of the motif bound by each type of binding molecule on the array can differ based on the inclusion, in each motif, of a different set of variable amino acids.

Usually the motif in each peptide or protein will contain three, four or five variable amino acids. These variable amino acids may be identified as part of the motif by virtue of their position within the peptide or protein (e.g. relative to the C-terminus, the N-terminus, or an internal feature) and/or by forming part of a larger motif that also contains 'constant' amino acids.

Additionally or alternatively a characteristic of the motif may be the presence of a modified amino acid, such as a phosphorylated amino acid or a glycosylated amino acid. Preferably, the motif should contain at least one unmodified amino acid. More preferably, all amino acids in the motif are unmodified.

### Sample fragmentation

The method of the invention may comprise the initial step of fragmenting the heterogeneous sample of proteins or peptides to produce a heterogeneous sample of peptide fragments.

Fragmentation of a heterogeneous sample of proteins or peptides can be advantageous because it can increase the number of peptide molecules representing each original protein or peptide. For example, if a protein in the original sample is fragmented, the binding of any one of its multiple fragments can be used as a marker or the presence and abundance of that protein. In other words, fragmentation increases the chances that any particular protein or peptide will be represented in any given heterogeneous class. This means that fewer binding molecules can be used without reducing the information that can be obtained from each sample analysed.

Fragmentation also allows for the detection of transmembrane proteins which, without fragmentation, cannot be analysed.

**Table 1**

| **Enzyme** | **Preferred Site** |
|---|---|
| trypsin: | R₁ = Lys, Arg |
| chymotrypsin | R₁ = Tyr, Phe, Leu, Ile, Val, Trp and His at high |
| | pH |
| pepsin | R₁ = Phe, Leu, many others |
| thrombin | R₁ = Arg |
| papain | R₁- Arg, Lys, Phe-X (CO side of residue next to |
| | Phe) |
| bromelain | R₁ = Lys, Ala, Tyr, Gly |
| *Staphylococcus* | R₁ = Glu, Asp |
| *aureus* protease | |
| Factor Xa | R₁ = Ile-Glu-Gly-Arg |
| thennolysin | R₂ = Tyr, Phe, Leu, Ile, Val, Trp and His |

Wherein R₁ and R₂ are defined according to the following formula:

N-terminal---NH-CHR₁-CO-NH-CHR₂-CO---C-terminal

The step of fragmenting of the heterogeneous sample of proteins, polypeptides or peptides may be achieved by any method known in the art. For example, chemical or enzymatic cleavage may be used. Numerous methods of chemical or enzymatic (i.e. protease directed) cleavage are known in the art. For example, proteases include trypsin, chymotrypsin, pepsin, thrombin, papain, bromelain, thermolysin, subsilisin, Factor Xa, *Staphylococcus aureus* protease and carboxypeptidase A. In a preferred embodiment, the fragmentation method will cleave proteins, polypeptides or peptides at defined locations. Enzymatic cleavage is typically sequence-directed, as shown in Table 1 above. Chemical cleavage methods may also be sequence-directed e.g. cyanogen bromide fragmentation, which will cleave a protein or peptide on the C-terminal side of methionine.

Thus, for example, trypsin cleavage is a sequence-directed means of fragmentation, since cleavage is directed by the presence of arginine or lysine residues in a protein, polypeptide or peptide, and accordingly produces cleavage fragments that have, as their C-terminal residue, either an arginine or lysine. The skilled person is aware of many other means of 'directed' fragmentation, such as those described in WO 02/25287.

Usually, the motif in each fragment will be at the same location in each fragment, relative to the site of cleavage. Thus, for example, where fragments are created by a sequence directed cleavage mechanism (see below), then the motif may comprise one or more amino acids adjacent to the site of the terminus created by cleavage, some of which may be constant as a result of the sequence directed cleavage mechanism.

Thus, one or more of the amino acids that form the sequence that directs the cleavage may be retained in the fragment. For example, where trypsin cleavage is used as the method of fragmentation then, the fragments produced have, as their C-terminal residue, either an arginine or lysine. Thus the motif may encompass amino acids forming part of the cleavage site.

Accordingly, the motif in each fragment generated may comprise one or more, such as two, three, four or more constant amino acids. For the purposes of the present invention, the skilled person will appreciate that term "constant", when used in the context of an amino acid within a motif, includes amino acids positions at which there is a low level of variability, such as 2, 3, 4, 5, 6, 7, 8, 9 or 10 different possibilities. Lower numbers are preferred. For example, the motif in tryptic fragments may comprise the C-terminal amino acid, which is thus a constant residue of either arginine or lysine. In other words, the identity of a "constant" amino acid is not as random as at other "variable" positions.

Thus, the motif may be formed from a mixture of constant and non-constant (i.e. variable) amino acids. Usually the motif will contain three, four or five variable amino acids, the other amino acids in the motif (if there are any), being constant between all fragments.

### Arrays

The step of separating the heterogeneous sample of proteins, peptides and/or fragments thereof into heterogeneous classes based on the presence of a motif is achieved by binding members of each class to a spaced apart defined location on an array.

Arrays *per se* are well known in the art. Typically they are formed of a linear or two-dimensional structure having spaced apart (i.e. discrete) regions ("spots"), each having a finite area, formed on the surface of a solid support. An array can also be a bead structure where each bead can be identified by a molecular code or colour code or identified in a continuous flow. Analysis can also be performed sequentially where the sample is passed over a series of spots each adsorbing the class of molecules from the solution. The solid support is typically glass or a polymer, the most commonly used polymers being cellulose, polyacrylamide, nylon, polystyrene, polyvinyl chloride or polypropylene. The solid supports may be in the form of tubes, beads, discs, silicon chips, microplates, polyvinylidene difluoride (PVDF) membrane, nitrocellulose membrane, nylon membrane, other porous membrane, non-porous membrane (e.g. plastic, polymer, perspex, silicon, amongst others), a plurality of polymeric pins, or a plurality of microtitre wells, or any other surface suitable for immobilising proteins, polynucleotides and other suitable molecules and/or conducting an immunoassay. The binding processes are well known in the art and generally consist of cross-linking covalently binding or physically adsorbing a protein molecule, polynucleotide or the like to the solid support. By using well-known techniques, such as contact or non-contact printing, masking or photolithography, the location of each spot can be defined. For reviews see Jenkins, R.E., Pennington, S.R. (2001, Proteomics, 2,13-29) and Lal et al (2002, Drug Discov Today 15;7(18 Suppl):S143-9).

Typically the array is a microarray. By "microarray" we include the meaning of an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance.

Typically the spots on the array comprises a number of different types of binding molecule (as defined below), each type being immobilised at a separate spot on the arrays. Thus by using a method of generating spots with defined locations, it is possible to know the identity and/or binding affinity of each spot on the array.

Preferably, each type of binding molecule, and therefore, each spot, is capable of binding specifically to a defined motif as defined above and the different types of binding molecule have different binding specificities. Thus proteins, peptides and/or fragments thereof that bind to one spot will share a common motif. Conversely, proteins, peptides and/or fragments thereof on different spots are separated into heterogeneous classes based on the presence of different motifs.

Thus, where the motif is a terminal sequence, such as a C-terminal sequence, then the binding molecule at one spot will bind specifically to a proteins, peptides and/or fragments thereof that comprises a given first C-terminal sequence, whereas a binding molecule at another spot will bind specifically to a proteins, peptides and/or fragments thereof that comprises a given second C-terminal sequence, the first and second C-terminal sequences being different.

In one embodiment, all binding molecules on the array are specific for C-terminal motifs. In another embodiment, all binding molecules on the array are specific for N-terminal motifs. In another embodiment, all binding molecules on the array are specific for motifs that are not positionally conserved.

Where the proteins or peptides are fragmented prior to analysis, then the defined target motifs may be selected dependent on the method of fragmentation used. For example, where trypsin cleavage is used as the method of fragmentation then, as discussed above, the fragments produced have, as their C-terminal residue, either an arginine or lysine. Thus, it may be useful to separate fragments based on, for example, their first four C-terminal resides. Since each fragment will have either an arginine or lysine as its C-terminal residue, then variability will be found only at positions 2, 3 and 4 (relative to the C-terminal residue that, in this context, is designated as position 1). In this example, the maximum level of variability displayed by the C-terminal tetrapeptide will be 2×20×20×20 = 16,000 different possible motifs. Using the same scheme, if the motif used to classify tryptic fragments is based on, for example, their first five C-terminal residues, then the maximum level of variability displayed will be 2×20×20×20×20 = 320,000 different possible motifs.

The skilled person will appreciate that the total number of different terminal motifs generated can be increased by increasing the number of variable amino acids in each motif target motif and decreased by replacing variable amino acids with constant amino acids. Moreover, the abundance of each motif in a heterogeneous sample of proteins, peptides or fragments thereof can be increased by reducing the size of the motif and decreased by increasing the size of the motif.

Thus, a method of fragmentation that uses a sequence-directed cleavage mechanism to generate fragments having a defined terminal amino acid or a defined terminal sequence can be used to reduce the total number of different terminal motifs, for any given length of motif.

An array suitable for use in a method as defined above may comprise a number of different types of binding molecule, each type immobilised at a defined and discrete location on the array, wherein each type of binding molecule is capable of binding specifically to a motif as defined above and wherein the different types of binding molecule have different binding specificities.

It is not necessary for the array to have as many different types of binding molecules as there are different possible motifs. This is because each binding molecule is specific only for a motif, not a particular protein (unlike the prior art methods, such as WO 02/25287), and so multiple different proteins, peptides of fragments thereof can bind to a given spot on the array. Moreover, where the protein or peptide sample is fragmented prior to analysis, then each protein or peptide in the original sample can generate multiple fragments. Thus the array may provide a suitable number of different types of binding molecule such that at least one fragment from each protein or peptide in the sample can bind specifically to a binding molecule.

In fact, the skilled person will appreciate that the heterogeneous sample of proteins or peptides may be usefully characterised even if not all proteins or peptides of the unfragmented sample can be represented. Ideally, the number of different types of binding molecule provided on an array is suitable to capture at least 10, 20, 30, 40, 50, 60, 70, 80, 90, 95, 98, 99 or substantially 100% of the types of proteins or peptides in the sample, or at least one fragment derived from the above stated percentage of types of proteins or peptides in a sample. The percentage as used herein refers not to the total protein content by mass, since a sample may comprise many different proteins but one particular protein may predominate and, in that case, the binding of the predominant protein to the exclusion of all others could represent capture of a high percentage of protein from the sample, yet would yield little or no proteomic information. Rather, percentage is used to reflect the variety of different proteinaceous species in the sample, irrespective of the abundance of each species. Thus each different type of protein or peptide in the unfragmented sample represents 'one' and the percentage capture of proteins or peptides from a sample can be determined by dividing the sum of all of the different types of captured proteins or peptides as determined by the method of the present invention by the sum of all of the different proteins or peptides fragments in the unfragmented sample as determined by methods known in the prior art such as two-dimensional electrophoresis coupled to mass spectrometry, and multiplying by one hundred.

As an *in silico* example, a simulated trypsin degradation of 10,000 protein sequences extracted from SwissProt results in 400,000 peptide fragments. The abundance of fragments having each type of possible C-terminal tetra peptide motif varies between 0-10 %. A suitable array may be formed by choosing binding molecules with affinity for suitably abundant motifs, and so a limited number of different binding molecules will be able to capture a large set of different fragments. For instance, as few as 200 different such binding molecules, each capturing on average 100 peptides, will capture 20,000 fragments from a tryptic digest of a protein preparation made from a tissue sample. *In silico* analysis of a theoretical proteome consisting of all human protein sequences in SwissProt (approximately 10,500 sequences) indicates that, if the motifs are randomly chosen from all possible motifs with a theoretical frequency of approximately 100 in the above defined proteome, the captured peptides would contain one or more peptide from 75% of all those proteins. A rational selection of binding molecules to avoid unnecessary overlap (by capturing many peptides from certain proteins and none from others) will increase the coverage further.

Accordingly, the array may have at least about 10, 50, 100, 150, 200, 250, 300, 350, 400, 500, 600, 700, 800, 900, 1000 or more different types binding molecules as defined above.

Each spot on the array may bind on average, 2, 4, 6, 8, 10, 20, 40, 60, 80, 100, 200, 400, 600, 800, 900, 1000, 1500, 2000 or more different types of proteins, peptides or fragments thereof, each having the same motif. In this context, "different types" of protein peptides or fragments thereof refers to protein peptides or fragments thereof that have at least one of the following: different sequences; different molecular masses; and/or different post-translational modifications.

### Binding Molecules

Binding molecules can be selected from a library, based on their ability to bind a given motif, as discussed below.

At least one type, more typically all of the types, of the binding molecules may be an antibody or fragments or variants thereof.

Thus, a fragment may contain one or more of the variable heavy (V_{H}) or variable light (V_{L}) domains. For example, the term antibody fragment includes Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544).

The term "antibody variant" includes any synthetic antibodies, recombinant antibodies or antibody hybrids, such as but not limited to, a single-chain antibody molecule produced by phage-display of immunoglobulin light and/or heavy chain variable and/or constant regions, or other immunointeractive molecule capable of binding to an antigen in an immunoassay format that is known to those skilled in the art.

A general review of the techniques involved in the synthesis of antibody fragments which retain their specific binding sites is to be found in Winter & Milstein (1991) Nature 349, 293-299.

Additionally or alternatively at least one type, more typically all of the types, of the binding molecules is an aptamer.

Additionally or alternatively at least one type, more typically all of the types, of the binding molecules is a polynucleotide.

### Selection of binding molecules

Molecular libraries such as antibody libraries (Clackson et al, 1991, Nature 352, 624-628; Marks et al, 1991, J Mol Biol 222(3): 581-97), peptide libraries (Smith, 1985, Science 228(4705): 1315-7), expressed cDNA libraries (Santi et al (2000) J Mol Biol 296(2): 497-508), libraries on other scaffolds than the antibody framework such as affibodies (Gunneriusson et al, 1999, Appl Environ Microbiol 65(9): 4134-40) or libraries based on aptamers (Kenan et al, 1999, Methods Mol Biol 118, 217-31) may be used as a source from which binding molecules that are specific for a given motif are selected for use in the methods of the invention.

The molecular libraries may be expressed *in vivo* in prokaryotic (Clackson *et al,* 1991, *op. cit.;* Marks *et al,* 1991, *op. cit.)* or eukaryotic cells (Kieke et al, 1999, Proc Natl Acad Sci USA, 96(10):5651-6) or may be expressed *in vitro* without involvement of cells (Hanes & Pluckthun, 1997, Proc Natl Acad Sci USA 94(10):4937-42; He & Taussig, 1997, Nucleic Acids Res 25(24):5132-4; Nemoto et al, 1997, FEBS Lett, 414(2):405-8).

In cases when protein based libraries are used often the genes encoding the libraries of potential binding molecules are packaged in viruses and the potential binding molecule is displayed at the surface of the virus (Clackson *et al,* 1991, *op. cit.;* Marks *et al,* 1991, *op. cit;* Smith, 1985, *op. cit.*).

The most commonly used such system, today, is filamentous bacteriophage displaying antibody fragments at their surfaces, the antibody fragments being expressed as a fusion to the minor coat protein of the bacteriophage (Clackson *et al,* 1991, *op. cit.;* Marks *et al,* 1991, *op. cit*). However, also other systems for display using other viruses (EP 39578), bacteria (Gunneriusson *et al,* 1999, *op. cit.;* Daugherty et al, 1998, Protein Eng 11(9):825-32; Daugherty et al, 1999, Protein Eng 12(7):613-21), and yeast (Shusta et al, 1999, J Mol Biol 292(5):949-56) have been used.

In addition, recently, display systems utilising linkage of the polypeptide product to its encoding mRNA in so called ribosome display systems (Hanes & Pluckthun, 1997, *op. cit.*; He & Taussig, 1997, *op. cit.;* Nemoto *et al,* 1997, *op. cit.*), or alternatively linkage of the polypeptide product to the encoding DNA (see US Patent No. 5,856,090 and WO 98/37186) have been presented.

When potential binding molecules are selected from libraries one or a few selector peptides having defined motifs are usually employed. Amino acid residues that provide structure, decreasing flexibility in the peptide or charged, polar or hydrophobic side chains allowing interaction with the binding molecule may be used in the design of motifs for selector peptides. For example -
(i) Proline may stabilise a peptide structure as its side chain is bound both to the alpha carbon as well as the nitrogen;
(ii) Phenylalanine, tyrosine and tryptophan have aromatic side chains and are highly hydrophobic, whereas leucine and isoleucine have aliphatic side chains and are also hydrophobic;
(iii) Lysine, arginine and histidine have basic side chains and will be positively charged at neutral pH, whereas aspartate and glutamate have acidic side chains and will be negatively charged at neutral pH;
(iv) Asparagine and glutamine are neutral at neutral pH but contain a amide group which may participate in hydrogen bonds;
(v) Serine, threonine and tyrosine side chains contain hydroxyl groups, which may participate in hydrogen bonds.

Typically selection of binding molecules may involve the use of array technologies and systems to analyse binding to spots corresponding to types of binding molecules.

Potential binding molecules, e.g. antibody fragments in a library, can be cloned and spotted in an array format. The position of the spot can correlate with the identity of the clone. Next, selector peptides having defined motifs would be allowed to bind to the array. To spots that happened to contain binding molecules against the defined motif of a particular selector peptide, that particular selector peptide binds, and binding gives a readable signal enabling the user to determine the position of the spot and, thus the identity of the clone from which the positive binding molecule was obtained. False positives (e.g. binding molecules that bind to regions of the selector peptide other than the motif) can be avoided by measuring the ability of putative positives to bind to similar peptides without the motif, wherein binding to these similar peptides indicates that the putative binder is a false positive.

Similarly, libraries of potential polynucleotide binding molecules can be screened for the ability to bind selector peptides having defined motifs (e.g. using the commercially available Affymetrix chip).

Once a suitable number of binding molecules have been isolated, the skilled person can manufacture an array.

A method for making a library of binding molecules may comprise the following steps -
(a) providing, as a first component, a selector peptide comprising a motif as defined above;
(b) providing, as a second component, a source of candidate binding molecules, such as a molecular library as defined above;
(c) combining the first and second components; and
(d) identifying candidate binding molecules that are capable of specifically binding to the motif of the selector peptide in the first component.

A library, typically a library wherein the members have been pre-selected by the above method may comprise at least about 10, 50, 100, 150, 200, 250, 300, or more different types of binding molecule, each type being capable of binding specifically to a motif as defined above and the different types having different binding specificities. At least one binding molecule in the library, usually all binding molecules in a library, may be antibodies or fragments or variants thereof, such as Fv, scFv or Fab; aptamers; and/or polynucleotides.

A library of binding molecules as defined above may be used to produce an array as described above.

A method for producing an array suitable for use in a method according to the first aspect of the present invention may comprise -
(a) providing a library of different types of binding molecule, each type being capable of binding specifically to a motif as defined above and the different types having different binding specificities; and
(b) immobilising the binding molecules on an array such that different types of binding molecule are immobilised at defined and discrete locations.

Methods of immobilising binding molecules such as antibodies, aptamers, polynucleotides and the like at defined and discrete locations on an array are discussed above, and in any case are well known in the art.

A system for analysing a heterogeneous sample of proteins or peptides may comprise an array as described above and a data carrier comprising information on the identity and/or binding property and position of each different type of binding molecule on the array. The data carrier may be an electronic data carrier, typically in the form of a computer-readable data carrier. The information may correlate position (spot) on the array with identity of a library clone that contributed the binding molecule at that array spot, thereby allowing the user to further investigate the characteristics of a binding molecule produced by a given clone. Additionally or alternatively, the data carrier may comprise information on the binding characteristics of a binding molecule at a given position on the array.

### Screening conditions

Having provided a suitable array, it is possible to analyse a sample according to the method of the invention. In order to separate a heterogeneous sample of proteins, peptides and/or fragments thereof into heterogeneous classes by binding each members of class to a spaced apart defined location on an array, each heterogeneous class having a motif common to that class, it is important for the binding conditions to be suitably stringent to substantially avoid non-specific binding.

The formation of binding moleclue:motif complexes can be performed under a variety of conditions. Peptide fragment-containing reaction solutions can contain varying degrees of salt or be presented at varying pH levels. In addition, the binding reaction can be carried out at varying temperatures. In general pH conditions will range from 2-10 (most preferably around pH 8), temperatures from 0°C -100°C and salt conditions from 1 µM to 5M (in the case of NaCl).

Following the step of combining the heterogeneous sample of proteins, peptides and/or fragments thereof with the array under conditions to that allow specific, the array is typically washed to remove unbound proteins, peptides or fragments thereof. Solutions appropriate for washing may contain salts, such as sodium chloride, buffering agents such as phosphate buffer, chaotropic agents such as urea and detergents such as Tween-20. The concentration of these components, as well as the pH of the solution, may be optimised to obtain suitably stringent washing condition. Prior to MALDI-TOF mass spectrometric analysis (see below), the array should be washed with distilled water to remove salts, detergents, polymers or other compounds that may interfere with the analysis.

The skilled person can adapt the binding reaction and wash conditions to arrive at an appropriate condition to avoid non-specific binding by applying a mixture of proteins, peptides and/or fragments thereof having known sequences to an array and determining whether any proteins, peptides and/or fragments thereof bind non-specifically (i.e. to spots having binding molecules of a type that are specific for a motif that is not contained in a proteins, peptides and/or fragments thereof of the mixture). If non-specific binding occurs, the stringency of the conditions used can be increased. Alternatively, the user can replace the binding molecule responsible for low specificity binding with a higher specificity binding molecule.

Affinity constants are a measure of the interaction between a particular ligand and its cognate receptor. The "binding affinity" or the measure of the strength of association between a particular binding molecule and its motif target is generally measured by affinity constants for the equilibrium concentrations of associated and dissociated configurations of the binding molecule and its target. Preferably the binding of a binding molecule to its motif should occur at an affinity of about K_{D}= 10⁻⁶M or greater to be useful for the present invention, with greater than about 10⁻⁷M being more preferable, and most preferably between about 10⁻⁸M and about 10⁻¹¹M. Antibody fragments will generally have binding affinities in the range of about 10⁻⁷M to 10⁻⁸M.

### Characterising heterogeneous classes of bound proteins, peptides and/or fragments thereof

Once separated into heterogeneous classes on an array, proteins, peptides or fragments thereof in each class may then be further characterised by analytical techniques known in the art such as desorption mass spectrometry (e.g. MALDI-TOF mass spectrometry; see Roepstorff, P, 2000, *EXS,* **88**:81-97), to yield information in the form of mass spectrograms, in which each peak will indicate the presence, mass and relative amount of a specific peptide.

Where fragmentation of the sample is performed prior to sample analysis, the identity of the protein or peptide from which the captured fragment is derived (i.e. the "parent protein") may be determined by collision induced dissociation mass spectrometry, which can be used to obtain structural information from a peptide.

Also, if the specificity of the binding molecule is known and sufficiently stringent conditions were used, one can know that a captured protein, peptide or fragment thereof on a given spot comprises a given motif. For example, if the motif is the first four C-terminal amino acids, then it is possible to deduce the sequence of the C-terminal tetra peptide of all proteins, peptides or fragments thereof at a given spot.

Information on motif content, in combination with accurate mass determination obtained by mass spectrometry, may be sufficient to match the information against a protein, peptide or fragment thereof generated by *in silico* analysis of a protein sequence database, or an *in silico* digestion of sequences present therein.

Accordingly, the step of characterising the proteins, peptides or fragments thereof in each heterogeneous class comprises characterising bound proteins, peptides or fragments thereof at each defined and discrete location on the array, by determining the mass of proteins, peptides or fragments thereof in each class and/or the abundance of each proteins, peptides or fragments thereof of different mass in each class. Usually this is performed by desorption mass spectrometry. The step of characterising the fragments in each heterogeneous class may additionally comprise determining the identity of the proteins or peptides in the unfragmented heterogeneous sample from which the detected fragments are derived (i.e. the "parents"). This is typically performed by collision induced mass spectrometry. The data thus acquired may yield sequence information or can be used to search protein sequence databases for matching sequences.

The relative intensity of the signal obtained from a specific peptide by mass spectrometry is dependent on the concentration, molecular weight and ionisation characteristics of the peptide. The quality of the quantification may be improved by addition of isotope-labelled reference proteins (Goshe B G and Smith D S (2003) Curr Opinion Biotech, 14:101-109).

Information regarding the abundance of a fragment and the identity of the parent protein or peptide may be used to quantify the parent protein or peptide in the unfragmented heterogeneous sample.

One of the benefits of the present invention can be seen in the analysis of each heterogeneous class of proteins, peptides or fragments thereof. The present invention provides for a method in which each heterogeneous class is analysed without the need for further separation of the components of each class. Thus the present invention has advantages over prior art methods which utilise multiple affinity separation steps (such as WO 02/060377), since the prior art methods rely on multiple peptide capture/elution steps and a complex fluid handling system, which are laborious and time-consuming. By contrast, the present invention provides a one-step method for subfractionation of proteins, peptides, or fragments thereof into different heterogenous classes followed by direct characterisation of each class, e.g. by mass spectrometry.

Additionally, the present invention provides qualitative and quantitative information about each heterogeneous class. For example, the molecular weight and abundance of each species within each class can be determined. This is an improvement over the prior art (e.g. WO 02/060377) which only provides for the determination of total amount of protein at any one spot.

### Applications

One application of the invention is for comparison between different samples. The skilled person will appreciate that the data generated by a method according to the present invention can be extremely complex and may involve several thousand different units of data. It may be appropriate to collect, store and analyse the data generated by electronic means. Therefore, a data carrier may comprise information obtainable by a method according to the first aspect of the present invention. An electronic data processing system, such as a computer, comprising a data carrier comprising information obtainable by a method according to the first aspect of the present invention and means for comparing information obtainable from the analysis of different samples may be provided. In this context, a means for comparing is typically a computer program designed to compare data generated from the analysis of a plurality of samples and highlight differences between the samples, thereby allowing the user to readily identify candidate proteins and peptides of interest.

Such comparisons may include samples from e.g. normal and diseased tissue or e.g. from tissues at various states of differentiation or activation. The invention can, thus, be used to rapidly and efficiently compare a large set of samples in order to search for differences in protein or peptide composition. Such differences may be used for identification of molecules with potential as drug targets.

Accordingly, a method of identifying differences in composition between two or more heterogeneous samples of proteins, polypeptides or peptides may comprise analysing each sample by a method according to the first aspect of the present invention, thereby to identify any differences.

An array or system as described above may be used to analyse one or more heterogeneous samples of proteins, peptides and/or fragments thereof, using methods as described above. The use may be to identify a disease-related protein by analysing at least one sample, typically an *ex vivo* sample, derived from an individual with the disease and at least one other sample, typically an *ex vivo* sample, derived from an individual without the disease. Suitable diseases for analysis include neurodegenerative diseases, cancer, inflammatory diseases, cardiovascular diseases and metabolic disorders.

Thus, a method for identifying a disease-related protein, polypeptide or peptide may comprise identifying differences between two or more samples by the above method, wherein at least one of the samples analysed is derived from an individual with the disease and another one of the samples analysed is derived from an individual without the disease.

Furthermore, once a disease-related protein or peptide has been identified, a method of diagnosing the disease state of an individual comprising analysing a sample, typically an *ex* vivo sample taken from the individual, by a method according to the first aspect of the present invention, and determining whether the results correspond with a disease-related protein, polypeptide or peptide identified by the method as described above can be provided.

Following diagnosis of an individual as having a disease or condition by using the above methods, that individual can be characterised as being in need of a treatment regime appropriate to the given condition diagnosed. Accordingly, a method of treating an individual identified as being in need thereof by a method of diagnosis as defined above may comprise administering an effective amount of a pharmaceutical agent appropriate to the disease state of the individual. Medical practitioners will be able to determine the effective amount of a pharmaceutical agent based on the patient's age, weight, gender and condition.

A pharmaceutical agent may be used in the manufacture of a medicament for treating an individual identified as being in need thereof by a method of diagnosis as defined above.

The invention will now be described in more detail by reference to the following non-limiting Figure and Examples wherein:

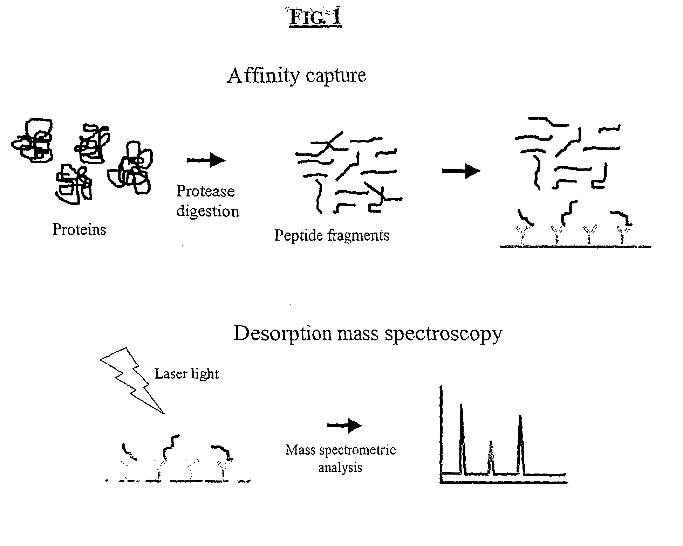

**Figure 1** shows a schematic overview of one embodiment of the present application.
**Figures 2-14** show mass spectra generated by analysis of tryptic peptide fragments bound to binding molecules selected for their abilities to bind to different C-terminal tetra or hexa peptides having either argine or lysine as the C-terminal residue.

### Example 1

This example describes how a microarray can be produced and used to detect peptides generated from a heterogeneous protein mixture. In this example, we choose to fragment the proteins into peptides by trypsin digestion and to capture sub-classes of peptide fragments using single chain Fv (scFv) molecules with binding properties directed towards the C-terminal of the peptides.

### Generation of binding molecules

Design of selector peptides: Synthetic peptides are used as catcher agents when isolating suitable single chain Fv (scFv) molecules from a phage-display library. The peptides are designed to capture phage particles displaying scFv with affinity to a C-terminal tetrapeptide in which the last (i.e. C-terminal) amino acid was either a lysine or and arginine. A spacer can be added on the N-terminal side of this tetrapeptide as well as an N-terminal biotin. The amino acid sequences are designed to include amino acids that are likely to generate good epitopes, such as hydrophobic amino acids (phenylalanine, tyrosine, tryptophan, leucine and isoleucine) or charged amino acids (aspartate, glutamate, asparagine, glutamine and histidine). Methionine is excluded due to its tendency to oxidise, and cysteine is excluded to avoid problems with dimerisation due to disulphide bridge formation. The sequences of the tetrapeptides are also decided based on their frequency in naturally occurring protein. Examples of suitable sequences are biotin-SGSG-XXXX-COOH where XXXX can be e.g. EDFR, EPER, HPDK, LPSR, LQSK, PEEK, WDSR or YLDK.

### Selection of specific binders from a phage display library.

The selection of specific binders from the n-CoDeR library can be performed using streptavidin coated magnetic beads (Hawkins, R.E., Russel, S.J. and Winter, G. (1992) J. Mol. Biol., 226, 889-896). The construction and handling of the n-CoDeR scFv phage display library is described in Söderlind et al (2000) Nature Biotech, 18, 852-856.

A volume containing 1-2×10¹³ CFU of the library phage-stock is mixed with biotinylated selector peptide (final concentration of peptide approx. 10⁻⁷ M). Add BSA to a final concentration of 3%, sodium azide to a final concentration of 0.02 % and Tween 20 to a final concentration of 0.05 %. Incubate at room temperature with gentle agitation for 1h. Add the magnetic beads (pre-blocked with albumin) and incubate for 15 minutes at room temperature with gentle agitation. Concentrate the beads with the magnet and remove the supernatant. Wash the beads with 3x1 ml 3% BSA, 0.05% Tween 20, 0.02% sodium azide in PBS, followed by 3x1 ml 0.05% Tween 20 in PBS and finally 3x1 ml PBS. Elute the binding phages by adding 400µl trypsin stock solution (1 mg/ml, Boehringer-Mannheim). Incubate for 30 minutes at room temperature. Transfer the eluate to a fresh tube and add 40µl aprotinin trypsin inhibitor stock solution (2 mg/ml). Determine the amount of phages in the eluate (by measuring the amount of CFU after infecting *E.coli*).

New scFv phage stocks are produced from the eluate by infecting logarithmically growing *E.coli* with the eluted phages. Add ampicillin to eliminate non-infected bacteria. The infected bacteria are amplified for approximately 3 hours, followed by infection with helper phages and IPTG induction for scFv displaying phage production. The selection cycle described above is repeated twice, but with an antigen concentration of 10⁻⁸ M for the second round and 10⁻⁹ M for the third. The resulting final eluate is stored a 4°C.

### Primary screening of binding molecules.

The selection process may generate tens of thousands of phage clones, including non-specific binders and specific binders of different quality. Also, not all clones will yield functional scFv. Phage pools eluted from the third selection are used to infect *E*. *coli* and plasmid (phagemid) DNA is isolated. Phage-specific DNA is eliminated by restriction enzyme digestion and re-ligated material is transformed into *E*. *coli.* Transformed, i.e. scFv expressing clones, are selected using ampicillin. To identify the clones that will generate the best binding molecules for the given application, a two-step screening procedure is employed. The primary screening is designed to evaluate the binding properties of a large number of expressed scFv (typically 10,000) against a predicted ligand and a predicted non-ligand, and will differentiate between scFv with specific *vs*. non-specific interaction with the selector peptide as well as providing a rough measure of relative quality between specific binders.

Primary screening is typically performed using automated, high-throughput systems for clone picking, expression and assay.

Typically 10,000 colonies are picked by a Qbot colony picker (Genetix; Hampshire, UK) and transferred to 384-well plates for individual growth over night. 5 µl of bacterial suspension is transferred (replicated) to Expression plates for growth and expression in an automated system (Thermo CRS; Burlington, Ontario, Canada).

In the ELISA system (Thermo CRS), assay plates are pre-coated with streptavidine (0.1 µg/well), incubated over night and washed. Plates are then coated with biotinylated peptides (1 pmole/well), incubated for 1 hour (or over night at +4°C), washed and blocked (block buffer: 0.45% Gelatine in 1xPBS with 0.05% Tween).

Supernatants from the expression plates are then added (10µl) to the assay plates and incubated for 1 hour, followed by a wash step.

A secondary antibody (mouse anti-his antibody conjugated with HRP) are then added and incubated for 1 hour, followed by a wash step.

Substrate (Pirce Supersignal ELISA Pico) is added followed by 10 min of incubation before reading in Luminescence mode.

Actives (clones with over 10 times ratio of ELISA signal between target and non target peptides) are cherry picked and retested (hit confirmation).

Specificity of clones is typically performed in a secondary screen where a larger set of peptides is tested. Selected hits with high specificity are then sequenced to obtain unique hit clones. Up to 96 hits are sequenced by colony PCR and dye termination cycle sequencing, using the ABI PRISM 3100 DNA Analyser (Applied Biosystems, Warrington, UK).

### Sequencing

Clones identified as specific binders during screening are analysed by DNA sequencing to identify unique clones.

The scFv encoding gene is sequenced according to the dideoxy-chain-terminating method using PCR amplified DNA as template, custom made primers and the Big Dye Terminator RR kit (Applied Biosystems, USA). Terminated fragments are separated and analysed using a 3100 Genetic Analyser (Applied Biosystems).

### Characterisation of ligands

A way to determine whether the scFv will actually capture a suitable number and type of peptides from a trypsin-digested sample is immunoaffinity extraction coupled to mass spectrometric analysis.

A sample containing plasma proteins is reduced (*e.g*. with mercaptoethanolamine), alkylated (*e.g*. with iodoacetamide), and digested with trypsin (20 µg trypsin/mg plasma protein, 6h incubation at 37°C).

The 6×His-tagged scFv can be captured on a small column (ZipTip^{™}, Millipore), prior modified with Ni²⁺ ions (protocol TN229, Millipore, USA). In principle, the immobilization of scFv selective to peptides from the trypsin-hydrolysed proteins of interest is performed by consecutive cycles of aspiration-dispension of an scFv solution (10-50 µg/ml in a neutral or slightly basic buffer, ≈10 µl) into the Ni-modified ZipTip^{™}. After removing the unbound scFv molecules, the antigens are captured into the affinity columns in a similar way as the one described above (*e.g*., by consecutive cycles of aspiration-dispension from ≈10 µl of the trypsin digest, previously diluted to a concentration of 2-3 mg protein/ml in PBS). After antigens trapping, the column is repeatedly washed to remove the unbound peptides. This washing step can be performed with PBS or, if a more stringent washing is required, with solution containing a higher salt (*e.g*., sodium chloride) concentration, denaturating agents (for example, guanidine or urea) or a detergent, such as Tween 20. The captured peptides are eluted in ≈1 µl elution medium (e.g. 5 % acetic acid or 50 % acetonitrile + 0.1 % trifluoroacetic acid (TFA)) directly onto a MALDI-TOF (matrix-assisted laser desorption/ionization - time-of-flight) target plate. Matrix solution (e.g., alpha-cyano-4-hydroxycinnamic acid, saturated in 1% TFA, 75 % acetonitrile) is then added on the top of each sample spot and allowed to dry. Alternatively, the matrix compound can be directly dissolved into the solution used for elution of peptides from the immunoextraction column.

The samples thus prepared are then analysed by MALDI-TOF mass spectrometry.

### Generation of affinity arrays.

The selected 6×His-tagged scFv are expressed in *E.coli,* dialysed and purified on a Ni-NTA column. After elution, the scFv are concentrated to 1-3 mg/ml in PBS. Then, scFv with different selectivity are spotted (using any of the current existing technology for protein spotting, for example non-contact or contact printing) on a suitable support (e.g., derivatised glass slides or well bottom of a microtiter plate). The scFv can be immobilized either covalently (e.g., via the reactive amino, aldehyde, or epoxy groups) on the surface of the support or non-covalently (for example, passive adsorption onto polystyrene or nitrocellulose-modified surfaces: for review, see Jenkins R.E. and Pennington, S.R. (2001) *Proteomics,* **1**, 13-29). Moreover, oriented immobilisation of scFv is possible, either via a Nichelate-modified glass slide able to bind to the 6×His tag, or by covalent coupling to maleimide-modified glass slides, binding covalently to a Cys tag, previously introduced in the scFv structure. The high throughput of the microarray can be exploited by spotting 1000-20000 different scFv on the same slide for simultaneous analysis of many antigens from the same sample. In this example, 200-300 different binding molecules may be sufficient, each spotted in duplicate or triplicate, giving a total number of spots of 400-1000. The arrays can be stored at 4°C for several weeks.

### Analysis of complex sample

Sample preparation: The sample to be analysed, e.g. plasma, can be directly trypsin digested after transfer to or dilution in a suitable buffer (e.g. 50 mM sodium bicarbonate, pH 7.0). Alternatively, the sample can be prefractionated to enrich proteins of interest or to remove certain components such as albumin and immunoglobulins (Anderson NL, Anderson NG. (2002) Mol Cell Proteomics, 1(11):845-67) to increase the limit of detection. The sample proteins may be reduced and carboxymethylated to avoid disulphide bridges between cysteine-containing peptides.

Sample application: 10-200 µl of the trypsin digested sample is applied on the printed microarray and incubated for 2 hours, either using an incubation chamber (Arrayit Hybridization Cassette, TeleChem International Inc, USA) or an automated sample processing instrument (e.g. ProteinArray Workstation, Perkin-Elmer, USA). Wash the microarray repeatedly with e.g. 50 mM phosphate buffer, pH 7.0, 0.1 % Tween, and 100 mM sodium chloride. For more stringent washing conditions, different salt or detergents can be added at various concentrations.

Detection: UV-absorbing matrix (alpha-cyano-4-hydroxycinnamic acid, saturated in 1% TFA, 75 % acetonitrile) is added to the array (100-500 nl/spot). The array is mounted onto a MALDI-TOF target plate (Borrebaeck CAK, Ekström S, Malmborg Hager AC, Nilsson J, Laurell T and Marko-Varga G (2001) Biotechniques 30, 1126-1132) and mass spectra from each spot are acquired using a MALDI-TOF mass spectrometer in reflector mode.

### Example 2

This example describes how an array of affinity columns can be produced and used to detect peptides generated from a heterogeneous protein mixture. In this example, we choose to fragment the proteins into peptides by trypsin digestion and to capture sub-classes of peptide fragments using single chain Fv (scFv) molecules with binding properties directed towards the C-terminal of the peptides.

### Generation of binding molecules

Design of selector peptides: Synthetic peptides were used as catcher agents when isolating suitable single chain Fv molecules from a phage-display library. The peptides were designed to capture phage particles displaying scFv with affinity to a C-terminal tetra or hexa peptide in which the last amino acid was either a lysine or arginine. A spacer was be added on the N-terminal side of this peptide as well as an N-terminal biotin. The amino acid sequences were designed to include amino acids that are likely to generate good epitopes, such as hydrophobic amino acids (phenylalanine, tyrosine, tryptophan, leucine and isoleucine) or charged amino acids (aspartate, glutamate, asparagine, glutamine and histidine). Methionine was excluded due to its tendency to oxidise, and cysteine was excluded to avoid problems with dimerisation due to disulphide bridge formation. The sequences of the peptides are also decided based on their frequency in naturally occurring proteins. The peptides used as selectors and competitors in this example are described in Table 2.

**Table 2. Peptides used during selection**

| **Name** | **Sequence** |
|---|---|
| FN1 | Biotin-SGSG-EDFR (-COOH) |
| FN2 | Biotin-SGSG-EPER (-COOH) |
| FN3 | Biotin-SGSG-EPFR (-COOH) |
| FN4 | Biotin-SGSG-HPDK (-COOH) |
| FN5 | Biotin-SGSG-LPSR (-COOH) |
| FN6 | Biotin-SGSG-LQSK (-COOH) |
| FN7 | Biotin-SGSG-PEEK (-COOH) |
| FN8 | Biotin-SGSG-TGEK (-COOH) |
| FN9 | Biotin-SGSG-WDSR (-COOH) |
| FN10 | Biotin-SGSG-YLDK (-COOH) |
| FN11 | SGSG-ASAK (-COOH) |
| FN12 | SGSG-ASAR (-COOH) |
| FN13 | Biotin-SGSG-LYEIAR (-COOH) |
| FN14 | Biotin-SGSG-DFAEDK (-COOH) |
| FN15 | Biotin-SGSG-LTEFAK (-COOH) |
| FN16 | Biotin-SGSG-TEEQLK (-COOH) |
| FN17 | Biotin-SGSG-SSAYSR (-COOH) |

### Selection of specific binders from a phage display library.

The selection of specific binders from the n-CoDeR library was performed using streptavidin coated magnetic beads (Hawkins, R.E., Russel, S.J. and Winter, G. (1992) J. Mol. Biol., 226, 889-896). The construction and handling of the n-CoDeR scFv phage display library is described in Söderlind et al (2000) Nature Biotech, 18, 852-856. Three consecutive rounds of selection were performed;Selection 1. The n-CoDeR^{™} phage library (Lib 2000) was first pre-selected against an irrelevant biotinylated peptide (biotin-GIVKYLYEDEG, 10⁻⁷ M). The peptide was captured on streptavidin magnetic beads and the beads were removed by centrifugation. This pre-selection removes binders against streptavidin, biotin and the SGSG linker.

The pre-selected phage stocks (one library equivalent per peptide pool) were selected against four pools of biotinylated peptides (5x10⁻⁸ M of each peptide). The composition of the pools was as shown in Table 3. Competitor peptides FN11 (10⁻⁶ M) and FN12 (10⁻⁶ M) were added to pools R and pools K, respectively.Table 3. Pools of target peptides used in selection 1

| **Tetra - Pool R** | **Tetra - Pool K** | **Hexa - Pool R** | **Hexa - Pool K** |
|---|---|---|---|
| FN1 | FN4 | FN 13 | FN14 |
| FN2 | FN6 | FN 17 | FN 15 |
| FN3 | FN7 | | FN 16 |
| FN5 | FN8 | | |
| FN9 | FN10 | | |

Peptides were captured on streptavidin magnetic beads and non-specific phages were removed by washing (beads were concentrated using a magnet). Phages bound to beads were eluted using trypsin and the eluted phage pools were amplified in *E*. *coli* HB101F'. Amplified phage stocks from selection 1 were pre-selected against an irrelevant peptide as described above. Pre-selected phage stocks were then used to selected binders to individual biotinylated peptides (2x10⁻⁸ M of each peptide). 15 separate selections were performed. This time both competitor peptides, FN11 and FN12 (2x10⁻⁷ M of each), were added to all selections.

Peptides were captured on streptavidin magnetic beads and non-specific phages were removed by washing. Phages bound to beads were eluted using acid. Eluted phage pools were not amplified but used directly in selection 3. Selection 3 was performed as a solid phase selection in 96 well ELISA plates. The eluted phage pools from selection 2 were first pre-selected against streptavidin (0.5 µg/well, 8 wells per selection) and then avidin (0.5 µg/well, 8 wells per selection).

Pre-selected phage stocks were used to select phages against target peptides loaded on avidin (10 pmol peptide/well, 8 wells per selection). Both competitor peptides (2x10⁻⁷ M of each) were added to all selections. Non-specific phages were removed by washing and phages bound to wells were eluted using trypsin.

The quality of the phage pools from selection 3 was evaluated in phage ELISA. The eluted phage pools were amplified in *E*. *coli* HB101F' and dilution series of amplified pools were tested against one target peptide and one non-target peptide.To identify the clones that will generate the best binding molecules for the given application, a two-step screening procedure was employed. The primary screening is designed to evaluate the binding properties of a large number of expressed scFv (typically 10,000) against a predicted ligand and a predicted non-ligand, and will differentiate between scFv with specific *vs*. non-specific interaction with the selector peptide as well as providing a rough measure of relative quality between specific binders.Based on the phage ELISA, the selections that showed enrichment of specific binders results were identified. Phage pools eluted from selection 3 were used to infect *E*. *coli* HB101F' and phagemid DNA was isolated. Phage-specific DNA was eliminated by restriction enzyme digestion and re-ligated material was transformed into chemically competent *E*. *coli* TOP10. Transformants, i.e. scFv expressing clones, were selected on LA plates containing ampicillin.

Single bacterial clones were picked and scFv was expressed in LB in 384-well plates for subsequent screening with luminescence ELISA (lum ELISA). 1920 colonies were picked for each target except FN9 (768 colonies) and FN15 (1008 colonies).

The lum ELISA screening was performer in 384-well format. Each scFv was screened against one target peptide and one non-target peptide. Biotinylated peptides (1 pmol/well) were loaded on streptavidin (0.1 µg/well) and detected using a HRP conjugated anti-His antibody.

All hexa-peptide selections (FN13-FN17) and three of the tetra-peptide selections (FN1, FN3, FN9) showed presence of specific scFv binders in the primary robot screening.

Clones identified as specific binders during screening were analysed by DNA sequencing to identify unique clones.

The scFv encoding genes were sequenced according to the dideoxy-chain-terminating method using PCR amplified DNA as template, custom made primers and the Big Dye Terminator RR kit (Applied Biosystems, USA). Terminated fragments were separated and analysed using a 3100 Genetic Analyser (Applied Biosystems).

To determine which scFv's will capture a suitable number and type of peptides from a trypsin-digested sample, the scFv's were coupled to a chromatography medium (Poros AL, Applied biosystems) and packed in gel loading tips to generate small affinity columns.

The samples were reduced with mercaptoethanolamine, alkylated with iodoacetamide, and digested with trypsin (PBS pH 7.4, 20 µg trypsin/mg protein, 6h incubation at 37°C). The affinity columns were used to capture peptides from trypsin-digested mouse liver homogenate, the captured peptides were eluted and analysed by matrix-assisted laser desorption/ionisation mass spectrometry.

### Generation of an affinity-column array.

14 scFv's were selected based on their ability to capture different subgroups of peptides from trypsinated mouse liver proteins. The coupling reaction of scFv's to POROS-AL chromatography medium (Applied Biosystems, Foster City, USA) was performed in accordance with the manufacturer's instructions. The slurry was packed in gel loading tips (Invitrogen) to generate affinity columns with a bed length of approximately 2 cm.

### Analysis of complex samples

Mouse liver homogenate was alkylated and fragmented as above and diluted 2 times in PBS pH 7.4. The affinity columns were washed with 2 x 10 µl 5 % acetic acid and equilibrated with 2 x 10 µl PBS pH 7.4. 10 µl of the sample was loaded onto the column followed by washing with 2 x 10 µl PBS pH 7.4. The column was eluted onto a Massprep MALDI target (Micromass, UK) with 7 µl 5 % acetic acid. The eluate was allowed to dry and the target well was washed twice with 0.1 % trifluoroacetic acid. Finally 1µl of 0.5 mg/ml α-Cyano-4-hydroxy-cinnamic acid in 75% acetonitrile / 1% trifluoroacetic acid was added. The samples were analysed using a Micromass M@ldi Reflectron mass spectrometer.

### Results

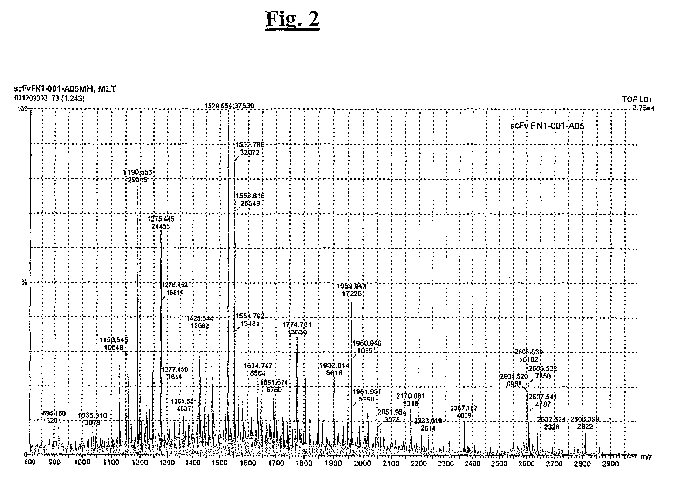

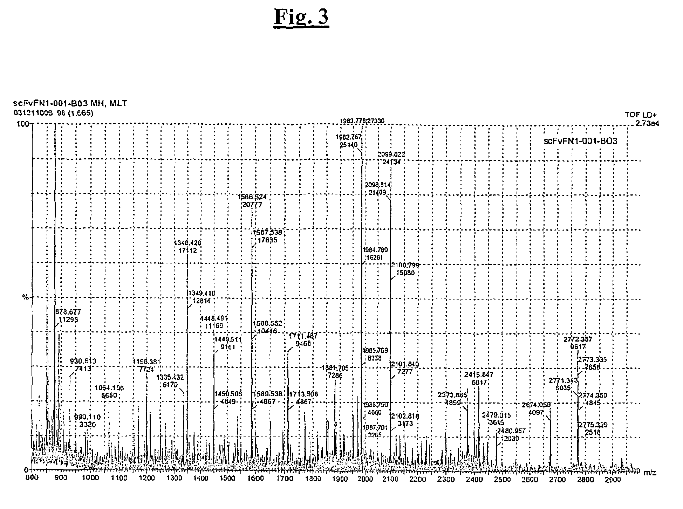

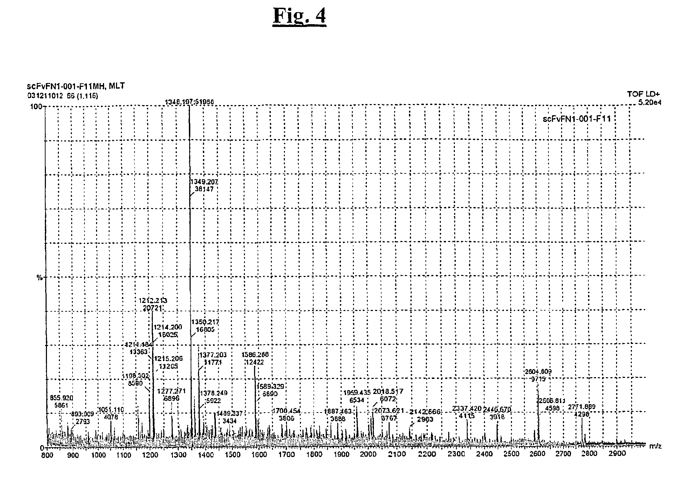

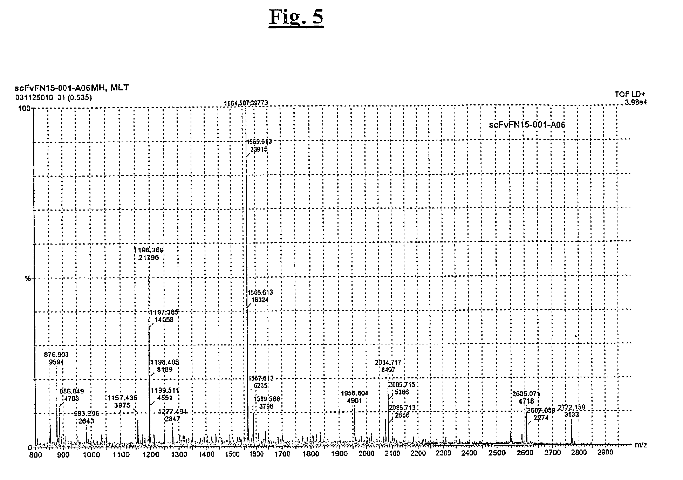

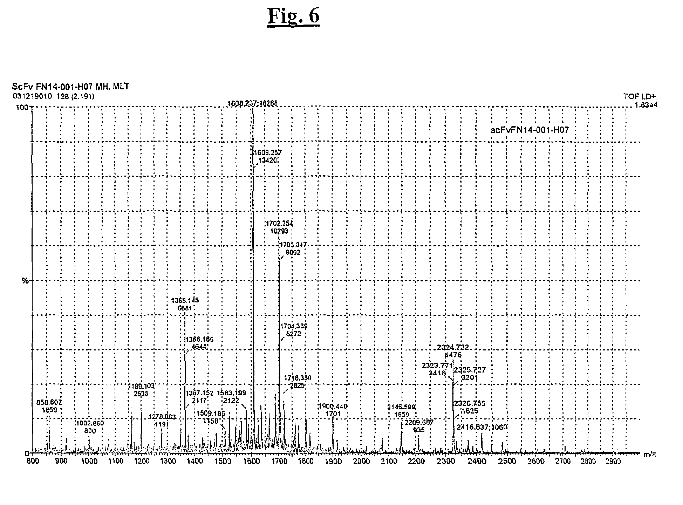

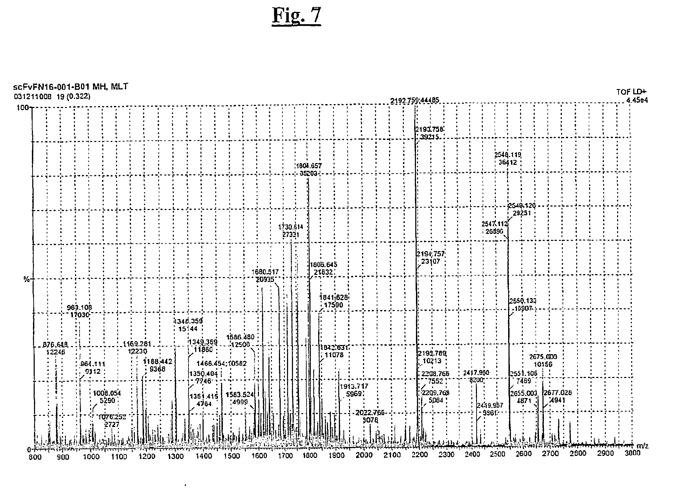

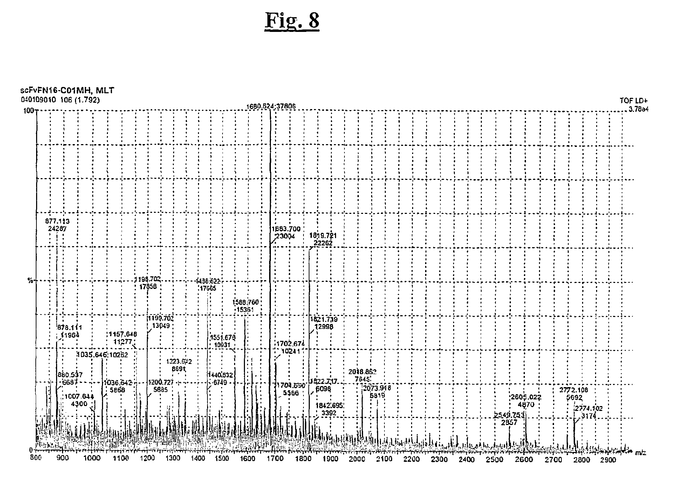

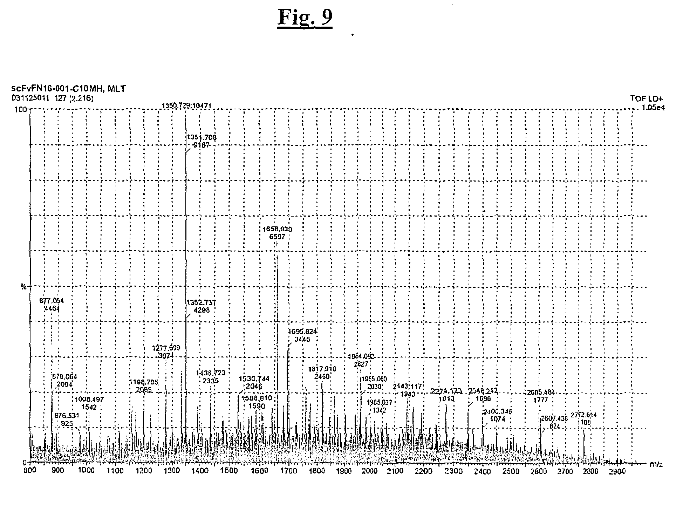

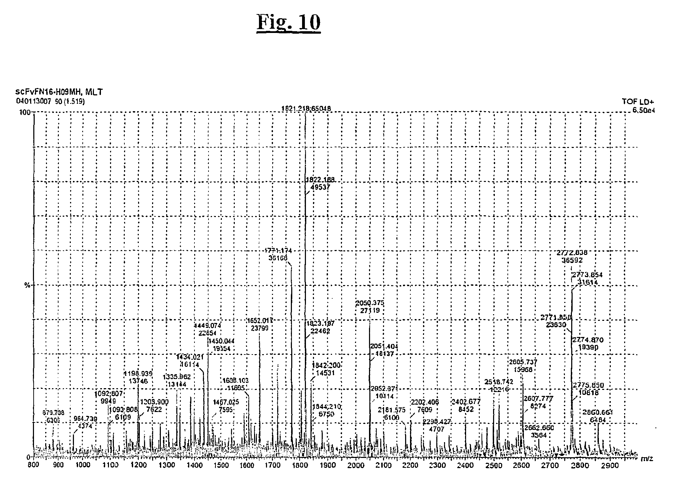

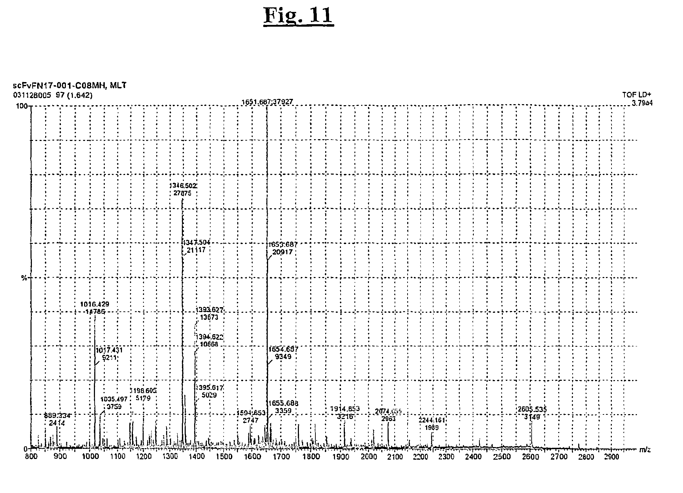

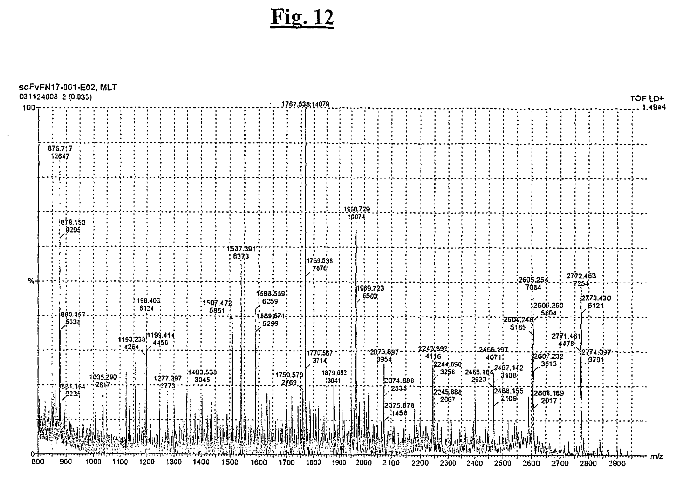

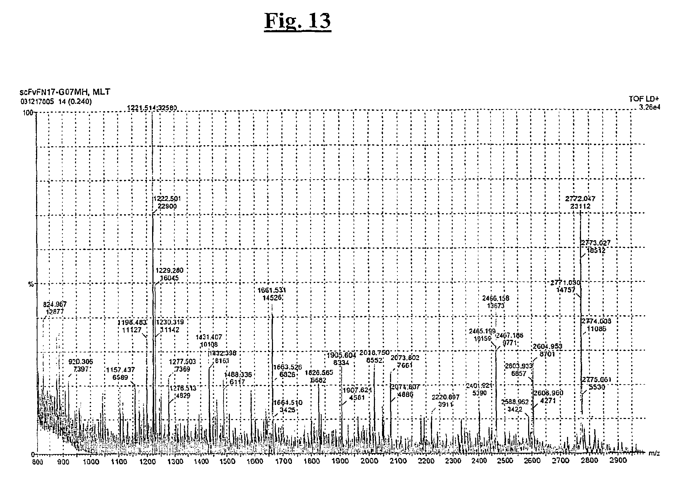

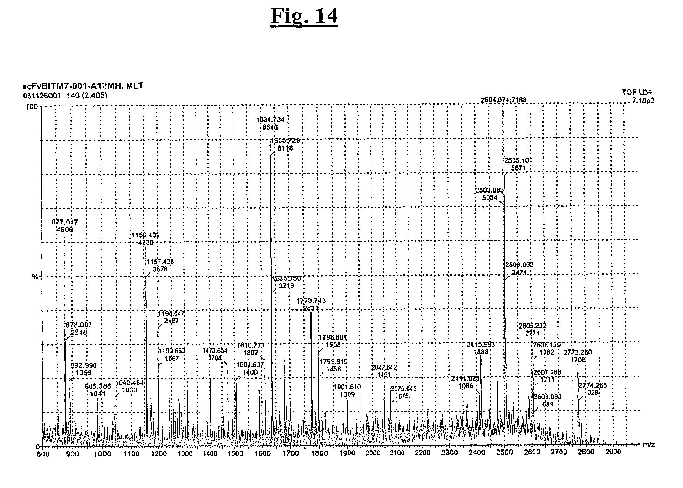

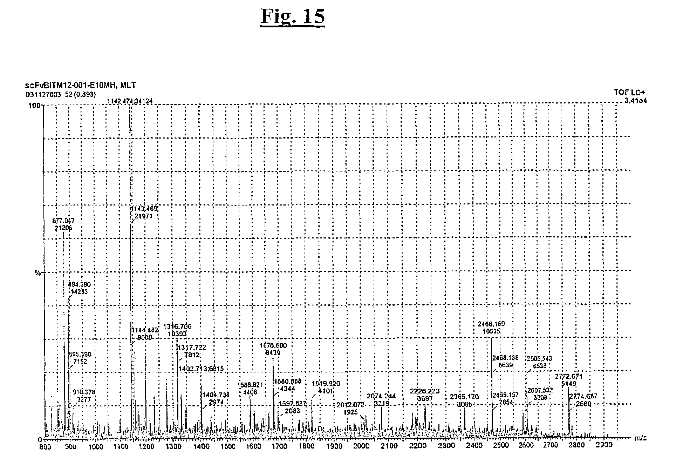

Figures 2-15 show the generated mass spectra. Each spectrum contain approximately 20-100 distinct peaks with, signal that has a signal-to-noise above 3, almost all peaks corresponding to a unique peptide. A few peaks can be detected in all spectra, these correspond to peptides that bind unspecifically to the Poros material. The total number of peptides that can be detected using this array is well above 500.

## Claims

1. A method for analysing a heterogeneous sample of peptides, or protein or peptide fragments, the method comprising -
(a) separating the heterogeneous sample of peptides, or protein or peptide fragments, into heterogeneous classes by binding members of each class to a spaced apart defined location on an array, wherein members of each class have a motif common to that class; and
(b) characterising the peptides, or protein or peptide fragments, in each class by determining the mass of peptides, or protein or peptide fragments, in the heterogeneous classes, and determining the abundance of peptides, or protein or peptide fragments, of different mass in the heterogeneous classes.

2. A method according to Claim 1 wherein the heterogeneous sample of peptides, or protein or peptide fragments, is an extract of the total protein content of a cell or tissue type.

3. A method according to Claim 1 or 2 wherein, prior to performing step (a) of Claim 1, the heterogeneous sample of fragments is formed by fragmenting a heterogeneous sample of proteins or peptides.

4. A method according to Claim 3 wherein the fragmenting is performed by chemical or enzymatic cleavage.

5. A method according to Claim 3 or 4 wherein the fragmenting is performed using a sequence-directed cleavage mechanism.

6. A method according to any one of Claim 3 to 5 wherein the fragmenting is performed by digestion of the heterogeneous sample of proteins or peptides with trypsin.

7. A method according to any preceding claim wherein the motif in each peptide, or protein or peptide fragment is at the same location in each peptide, or protein or peptide fragment, relative to the C-terminus, the N-terminus, or an internal feature.

8. A method according to any preceding claim wherein the sample is a heterogeneous sample of fragments of proteins or peptides and the motif in each fragment is at the same location in each fragment, relative to the site of cleavage.

9. A method according to any preceding claim wherein the motif in each peptide, or protein or peptide fragment, is three, four, live, six or more amino acids in length.

10. A method according to any preceding claim wherein the motif contains three, four or five variable amino acids, the other amino acids in the motif being constant between all peptides, or protein or peptide fragments.

11. A method according to any preceding claim wherein the motif is at the C-terminus.

12. A method according to one of Claims 1 to 10 wherein the motif is at the N-terminus.

13. A method according to any preceding claim wherein the array comprises a number of different types of binding molecule, each type immobilised at a spaced apart defined location on the array, wherein each type of binding molecule is capable of binding specifically to a defined motif as defined in any preceding claim and wherein different types of binding molecule have different binding specificities.

14. A method according to Claim 13 wherein the number of different types of binding molecule provided on the array is suitable to capture at least 10% of peptides in the unfragmented sample or, where the sample is a heterogeneous sample of fragments of proteins or peptides, at least one fragment from at least 10% of the proteins or peptides in the unfragmented sample.

15. A method according to Claim 13 wherein the number of different types of binding molecule provided on the array is suitable to capture at least 50% of peptides in the unfragmented sample or, where the sample is a heterogeneous sample of fragments of proteins or peptides, at least one fragment from at least 50% of the proteins or peptides in the unfragmented sample.

16. A method according to Claim 13 wherein the number of different types of binding molecule provided on the array is suitable to capture substantially 100% of peptides in the unfragmented sample or, where the sample is a heterogeneous sample of fragments of proteins or peptides, at least one fragment from substantially 100% of the proteins or peptides in the unfragmented sample.

17. A method according to any one of Claims 13 to 16 wherein the array has at least about 10, 50, 100, 150, 200, 250, 300, or more different types of binding molecules provided thereon.

18. A method according to any one of Claims 13 to 17 wherein at least one type of the binding molecules is an antibody or a fragment or variant thereof, such as Fv, scFv or Fab.

19. A method according to any one of Claims 13 to 18 wherein at least one of the types of the brinding molecules is an aptamer.

20. A method according to any one of Claims 13 to 19 wherein at least one of the types of the binding molecules is a polynucleotide.

21. A method according to any one of the previous claims wherein step (b) of Claim 1 comprises characterising bound peptides, or protein or peptide fragments, at the defined and discrete locations on the array.

22. A method according to any one of the preceding claims wherein step (b) of Claim 1 comprises characterising the peptides, or protein or peptide fragments, in the heterogeneous classes by desorption mass spectrometry or collision induced dissociation mass spectrometry.

23. A method according to any preceding claim wherein the information derived from step (b) of Claim 1 is used to determine the identity of the parent protein or peptide in the unfragmented heterogeneous sample from which a detected protein or peptide fragment is derived.

24. A method according to any preceding claim wherein the information derived from step (b) of Claim 1 is used to determine the abundance of the parent protein or peptide in the unfragmented heterogeneous sample from which a detected protein or peptide fragment is derived.

25. A method for identifying differences in composition between two or more heterogeneous fragmented or unfragmented samples of peptides, or protein or peptide fragments, comprising analysing each sample by the method according to any one of the preceding claims and comparing the results, thereby to identify any differences.

26. A method for identifying a disease-related protein or peptide comprising identifying differences between two or more samples by the method of Claim 25, wherein at least one of the samples analysed is derived from an individual with the disease and another one of the samples analysed is derived from a individual without the disease.

27. A method for analysing a heterogenous sample of peptides, or protein or peptide fragments, the method comprising.
(a) providing, as a first component, a selector peptide comprising a motif as defined in any one of Claims 7 to 12;
(b) providing, as a second component, a source of candidate binding molecules;
(c) combining the first and second components;
(d) identifying candidate binding molecules that are capable of specifically binding to the motif of the selector peptide in the first component;
(e) immobilising the binding molecules identified in step (d) on an array such that different types of binding molecule are immobilised at defined and discrete locations
(f) providing a heterogeneous sample of peptides, or protein or peptide fragments, which sample comprises peptides, or protein or peptide fragments, each having a motif that is bound by a binding molecule immobilised in step (e);
(g) separating the heterogeneous sample of peptides, or protein or peptide fragments, into heterogeneous classes by binding members of each class to the binding molecules immobilised in step (e); and
(h) characterising the peptides, or protein or peptide fragments, in each class by determining the mass of peptides, or protein or peptide fragments, in the heterogeneous classes, and determining the abundance of peptides, or protein or peptide fragments, of different mass in the heterogeneous classes.

## Patentansprüche

1. Verfahren zur Analyse einer heterogenen Probe von Peptiden oder Protein- oder Peptidfragmenten, wobei das Verfahren umfasst:
(a) Trennen der heterogenen Probe von Peptiden oder Protein- oder Peptidfragmenten in heterogene Klassen durch Binden von Elementen jeder Klasse an eine beabstandete definierte Stelle auf einem Array, wobei Elemente jeder Klasse ein Motiv aufweisen, das dieser Klasse gemein ist; und
(b) Charakterisieren der Peptide oder Protein- oder Peptidfragmente in jeder Klasse durch Bestimmen der Masse der Peptide oder Protein- oder Peptidfragmente in den heterogenen Klassen, und Bestimmen der Menge von Peptiden oder Protein- oder Peptidfragmenten unterschiedlicher Masse in den heterogenen Klassen.

2. Verfahren nach Anspruch 1, wobei die heterogene Probe von Peptiden oder Protein- oder Peptidfragmenten ein Extrakt des gesamten Proteingehalts einer Zellen- oder Gewebeart ist.

3. Verfahren nach Anspruch 1 oder 2, wobei vor der Durchführung von Schritt (a) von Anspruch 1 die heterogene Probe von Fragmenten durch Fragmentieren einer heterogenen Probe von Proteinen oder Peptiden gebildet wird.

4. Verfahren nach Anspruch 3, wobei die Fragmentierung durch chemische oder enzymatische Spaltung ausgeführt wird.

5. Verfahren nach Anspruch 3 oder 4, wobei die Fragmentierung unter Verwendung eines sequenzgerichteten Spaltungsmechanismus ausgeführt wird.

6. Verfahren nach einem der Ansprüche 3 bis 5, wobei die Fragmentierung durch Aufschluss der heterogenen Probe von Proteinen oder Peptiden mit Trypsin durchgeführt wird.

7. Verfahren nach einem der vorangehenden Ansprüche, wobei das Motiv in jedem Peptid oder Protein- oder Peptidfragment in jedem Peptid oder Protein- oder Peptidfragment an derselben Stelle in Bezug auf den C-Terminus, den N-Terminus oder ein internes Merkmal ist.

8. Verfahren nach einem der vorangehenden Ansprüche, wobei die Probe eine heterogene Probe von Fragmenten von Proteinen oder Peptiden ist und das Motiv in jedem Fragment an derselben Stelle in jedem Fragment relativ zu der Spaltungsstelle liegt.

9. Verfahren nach einem der vorangehenden Ansprüche, wobei das Motiv in jedem Peptid oder Protein- oder Peptidfragment eine Länge von drei, vier, fünf, sechs oder mehr Aminosäuren aufweist.

10. Verfahren nach einem der vorangehenden Ansprüche, wobei das Motiv drei, vier oder fünf variable Aminosäuren enthält, während die anderen Aminosäuren in dem Motiv zwischen allen Peptiden oder Protein- oder Peptidfragmenten konstant sind.

11. Verfahren nach einem der vorangehenden Ansprüche, wobei sich das Motiv am C-Terminus befindet.

12. Verfahren nach einem der Ansprüche 1 bis 10, wobei sich das Motiv am N-Terminus befindet.

13. Verfahren nach einem der vorangehenden Ansprüche, wobei das Array eine Anzahl verschiedener Arten von Bindungsmolekül umfasst, wobei jede Art an einer beabstandeten definierten Stelle auf dem Array immobilisiert ist, wobei jede Art von Bindungsmolekül imstande ist, spezifisch an ein definiertes Motiv zu binden, wie in einem der vorangehenden Ansprüche definiert, und wobei verschiedene Arten von Bindungsmolekülen verschiedene Bindungsspezifitäten haben.

14. Verfahren nach Anspruch 13, wobei die Anzahl verschiedener Arten von Bindungsmolekül, die auf dem Array bereitgestellt ist, geeignet ist, mindestens 10% von Peptiden in der nicht fragmentierten Probe zu erfassen, oder, wenn die Probe eine heterogene Probe von Fragmenten von Proteinen oder Peptiden ist, mindestens ein Fragment von mindestens 10% der Proteine oder Peptide in der nicht fragmentierten Probe zu erfassen.

15. Verfahren nach Anspruch 13, wobei die Anzahl verschiedener Arten von Bindungsmolekül, die auf dem Array bereitgestellt ist, geeignet ist, mindestens 50% von Peptiden in der nicht fragmentierten Probe zu erfassen, oder, wenn die Probe eine heterogene Probe von Fragmenten von Proteinen oder Peptiden ist, mindestens ein Fragment von mindestens 50% der Proteine oder Peptide in der nicht fragmentierten Probe zu erfassen.

16. Verfahren nach Anspruch 13, wobei die Anzahl verschiedener Arten von Bindungsmolekül, die auf dem Array bereitgestellt ist, geeignet ist, im Wesentlichen 100% von Peptiden in der nicht fragmentierten Probe zu erfassen, oder, wenn die Probe eine heterogene Probe von Fragmenten von Proteinen oder Peptiden ist, mindestens ein Fragment von im Wesentlichen 100% der Proteine oder Peptide in der nicht fragmentierten Probe zu erfassen.

17. Verfahren nach einem der Ansprüche 13 bis 16, wobei auf dem Array mindestens etwa 10, 50, 100, 150, 200, 250, 300 oder mehr verschiedene Arten von Bindungsmolekülen bereitgestellt sind.

18. Verfahren nach einem der Ansprüche 13 bis 17, wobei mindestens eine Art der Bindungsmoleküle ein Antikörper oder ein Fragment oder eine Variante davon ist, wie Fv, scFv oder Fab.

19. Verfahren nach einem der Ansprüche 13 bis 18, wobei mindestens eine der Arten der Bindungsmoleküle ein Aptamer ist.

20. Verfahren nach einem der Ansprüche 13 bis 19, wobei mindestens eine der Arten der Bindungsmoleküle ein Polynucleotid ist.

21. Verfahren nach einem der vorangehenden Ansprüche, wobei der Schritt (b) von Anspruch 1 das Charakterisieren gebundener Peptide oder Protein- oder Peptidfragmente an den definierten und getrennten Stellen auf dem Array umfasst.

22. Verfahren nach einem der vorangehenden Ansprüche, wobei der Schritt (b) von Anspruch 1 das Charakterisieren gebundener Peptide oder Protein- oder Peptidfragmente in den heterogenen Klassen durch Desorption-Massenspektrometrie oder durch kollisionsinduzierte Dissoziation-Massenspektrometrie umfasst.

23. Verfahren nach einem der vorangehenden Ansprüche, wobei die Informationen, die von Schritt (b) von Anspruch 1 abgeleitet werden, zur Bestimmung der Identität des Elternproteins oder -peptids in der nicht fragmentierten, heterogenen Probe verwendet werden, aus der ein nachgewiesenes Protein- oder Peptidfragment abgeleitet wird.

24. Verfahren nach einem der vorangehenden Ansprüche, wobei die Informationen, die von Schritt (b) von Anspruch 1 abgeleitet werden, zur Bestimmung der Menge des Elternproteins oder -peptids in der nicht fragmentierten, heterogenen Probe verwendet werden, aus der ein nachgewiesenes Protein- oder Peptidfragment abgeleitet wird.

25. Verfahren zur Identifizierung von Unterschieden in der Zusammensetzung zwischen zwei oder mehr heterogenen, fragmentierten oder nicht fragmentierten Proben von Peptiden oder Protein- oder Peptidfragmenten, umfassend die Analyse jeder Probe durch das Verfahren nach einem der vorangehenden Ansprüche und das Vergleichen der Ergebnisse, wodurch Unterschiede identifiziert werden.

26. Verfahren zur Identifizierung eines mit einer Krankheit in Zusammenhang stehenden Proteins oder Peptids, umfassend die Identifizierung von Unterschieden zwischen zwei oder mehr Proben durch das Verfahren von Anspruch 25, wobei mindestens eine der analysierten Proben von einem Probanden mit einer Krankheit abgeleitet ist, und eine andere der analysierten Proben von einem Probanden ohne Krankheit abgeleitet ist.

27. Verfahren zur Analyse einer heterogenen Probe von Peptiden oder Protein- oder Peptidfragmenten, wobei das Verfahren umfasst:
(a) Bereitstellen eines Selektor-Peptids als erste Komponente, umfassend ein Motiv wie in einem der Ansprüche 7 bis 12 definiert;
(b) Bereitstellen einer Quelle von Kandidat-Bindungsmolekülen als zweite Komponente;
(c) Kombinieren der ersten und zweiten Komponente;
(d) Identifizieren von Kandidat-Bindungsmolekülen, die imstande sind, spezifisch an das Motiv des Selektor-Peptids in der ersten Komponente zu binden;
(e) Immobilisieren der Bindungsmoleküle, die in Schritt (d) identifiziert wurden, auf einem Array, so dass verschiedene Arten von Bindungsmolekülen an definierten und getrennten Stellen immobilisiert sind;
(f) Bereitstellen einer heterogenen Probe von Peptiden oder Protein- oder Peptidfragmenten, wobei die Probe Peptide oder Protein- oder Peptidfragmente umfasst, die jeweils ein Motiv aufweisen, das durch ein Bindungsmolekül gebunden ist, das in Schritt (e) immobilisiert wurde;
(g) Trennen der heterogenen Proben von Peptiden oder Protein- oder Peptidfragmenten in heterogene Klassen durch Binden von Elementen jeder Klasse an die Bindungsmoleküle, die in Schritt (e) immobilisiert wurden; und
(h) Charakterisieren der Peptide oder Protein- oder Peptidfragmente in jeder Klasse durch Bestimmen der Masse der Peptide oder Protein- oder Peptidfragmente in den heterogenen Klassen, und Bestimmen der Menge von Peptiden oder Protein- oder Peptidfragmenten unterschiedlicher Masse in den heterogenen Klassen.

## Revendications

1. Procédé d'analyse d'un échantillon hétérogène de peptides, ou de fragments protéiques ou peptidiques, le procédé comprenant :
(a) la séparation de l'échantillon hétérogène de peptides, ou de fragments protéiques ou peptidiques, en classes hétérogènes par liaison des membres de chaque classe à un emplacement défini et espacé par rapport aux autres emplacements sur une matrice, dans lequel les membres de chaque classe ont un motif commun à cette classe ; et
(b) la caractérisation des peptides, ou des fragments protéiques ou peptidiques dans chaque classe par détermination de la masse des peptides, ou des fragments protéiques ou peptidiques dans les classes hétérogènes, et par détermination de l'abondance des peptides, ou des fragments protéiques ou peptidiques de masse différente dans les classes hétérogènes.

2. Procédé selon la revendication 1, dans lequel l'échantillon hétérogène de peptides, ou de fragments protéiques ou peptidiques, est un extrait de la teneur totale en protéines d'un type cellulaire ou tissulaire.

3. Procédé selon la revendication 1 ou 2, dans lequel, avant l'exécution de l'étape (a) de la revendication 1, l'échantillon hétérogène de fragments est formé par fragmentation d'un échantillon hétérogène de protéines ou de peptides.

4. Procédé selon la revendication 3, dans lequel la fragmentation est exécutée par clivage chimique ou enzymatique.

5. Procédé selon la revendication 3 ou 4, dans lequel la fragmentation est exécutée en utilisant un mécanisme de clivage dirigé par séquences.

6. Procédé selon l'une quelconque des revendications 3 à 5, dans lequel la fragmentation est exécutée par digestion de l'échantillon hétérogène de protéines ou de peptides avec la trypsine.

7. Procédé selon l'une quelconque des revendications précédentes, dans lequel le motif dans chaque peptide, ou fragment protéique ou peptidique, se trouve au même emplacement dans chaque peptide, ou fragment protéique ou peptidique, par rapport à l'extrémité C-terminale, à l'extrémité N-terminale, ou à une particularité interne.

8. Procédé selon l'une quelconque des revendications précédentes, dans lequel l'échantillon est un échantillon hétérogène de fragments de protéines ou de peptides et le motif dans chaque fragment se trouve au même emplacement dans chaque fragment, par rapport au site de clivage.

9. Procédé selon l'une quelconque des revendications précédentes, dans lequel le motif dans chaque peptide, ou fragment protéique ou peptidique, a une longueur de trois, quatre, cinq, six acides aminés ou davantage.

10. Procédé selon l'une quelconque des revendications précédentes, dans lequel le motif contient trois, quatre ou cinq acides aminés variables, les autres acides aminés dans le motif étant constants parmi tous les peptides, ou fragments protéiques ou peptidiques.

11. Procédé selon l'une quelconque des revendications précédentes, dans lequel le motif se trouve au niveau de l'extrémité C-terminale.

12. Procédé selon l'une quelconque des revendications 1 à 10, dans lequel le motif se trouve au niveau de l'extrémité N-terminale.

13. Procédé selon l'une quelconque des revendications précédentes, dans lequel la matrice comprend un certain nombre de types différents de molécules de liaison, chaque type étant immobilisé au niveau d'un emplacement défini et espacé par rapport aux autres emplacements sur la matrice, dans lequel chaque type de molécules de liaison est capable de se lier spécifiquement à un motif défini tel que défini dans l'une quelconque des revendications précédentes et dans lequel des types différents de molécules de liaison ont des spécificités de liaison différentes.

14. Procédé selon la revendication 13, dans lequel le nombre des différents types de molécules de liaison prévus sur la matrice est adapté pour capturer au moins 10 % des peptides dans l'échantillon non fragmenté ou, lorsque l'échantillon est un échantillon hétérogène de fragments de protéines ou de peptides, au moins un fragment provenant d'au moins 10 % des protéines ou des peptides dans l'échantillon non fragmenté.

15. Procédé selon la revendication 13, dans lequel le nombre des différents types de molécules de liaison prévus sur la matrice est adapté pour capturer au moins 50 % des peptides dans l'échantillon non fragmenté ou, lorsque l'échantillon est un échantillon hétérogène de fragments de protéines ou de peptides, au moins un fragment provenant d'au moins 50 % des protéines ou des peptides dans l'échantillon non fragmenté.

16. Procédé selon la revendication 13, dans lequel le nombre des différents types de molécules de liaison prévus sur la matrice est adapté pour capturer sensiblement 100 % des peptides dans l'échantillon non fragmenté ou, lorsque l'échantillon est un échantillon hétérogène de fragments de protéines ou de peptides, au moins un fragment provenant de sensiblement 100 % des protéines ou des peptides dans l'échantillon non fragmenté.

17. Procédé selon l'une quelconque des revendications 13 à 16, dans lequel la matrice présente au moins environ 10, 50, 100, 150, 200, 250, 300, ou davantage de types différents de molécules de liaison prévus sur celle-ci.

18. Procédé selon l'une quelconque des revendications 13 à 17, dans lequel au moins un type de molécules de liaison est un anticorps ou un fragment ou une variante de celui-ci, comme Fv, scFv ou Fab.

19. Procédé selon l'une quelconque des revendications 13 à 18, dans lequel au moins l'un des types de molécules de liaison est un aptamère.

20. Procédé selon l'une quelconque des revendications 13 à 19, dans lequel au moins l'un des types de molécules de liaison est un polynucléotide.

21. Procédé selon l'une quelconque des revendications précédentes, dans lequel l'étape (b) de la revendication 1 comprend la caractérisation des peptides, ou des fragments protéiques ou peptidiques liés au niveau des emplacements définis et discrets sur la matrice.

22. Procédé selon l'une quelconque des revendications précédentes, dans lequel l'étape (b) de la revendication 1 comprend la caractérisation des peptides, ou des fragments protéiques ou peptidiques, dans les classes hétérogènes par spectrométrie de masse par désorption ou par spectrométrie de masse par dissociation induite par collision.

23. Procédé selon l'une quelconque des revendications précédentes, dans lequel les informations obtenues de l'étape (b) de la revendication 1 sont utilisées pour déterminer l'identité de la protéine ou du peptide parent dans l'échantillon hétérogène non fragmenté à partir duquel un fragment protéique ou peptidique détecté est obtenu.

24. Procédé selon l'une quelconque des revendications précédentes, dans lequel les informations obtenues de l'étape (b) de la revendication 1 sont utilisées pour déterminer l'abondance de la protéine ou du peptide parent dans l'échantillon hétérogène non fragmenté à partir duquel un fragment protéique ou peptidique détecté est obtenu.

25. Procédé d'identification de différences de composition entre deux ou davantage échantillons fragmentés ou non fragmentés hétérogènes de peptides, ou de fragments protéiques ou peptidiques, comprenant l'analyse de chaque échantillon par le procédé selon l'une quelconque des revendications précédentes et la comparaison des résultats, afin d'identifier ainsi toutes les différences.

26. Procédé d'identification d'une protéine ou d'un peptide lié à une maladie comprenant l'identification de différences entre deux échantillons ou davantage par le procédé selon la revendication 25, dans lequel au moins l'un des échantillons analysés provient d'un individu atteint de la maladie et un autre des échantillons analysés provient d'un individu sans la maladie.

27. Procédé d'analyse d'un échantillon hétérogène de peptides, ou de fragments protéiques ou peptidiques, le procédé comprenant :
(a) la fourniture, comme premier composant, d'un peptide sélecteur comprenant un motif tel que défini dans l'une quelconque des revendications 7 à 12 ;
(b) la fourniture, comme second composant, d'une source de molécules de liaison candidates ;
(c) la combinaison des premier et second composants ;
(d) l'identification des molécules de liaison candidates qui sont capables de se lier spécifiquement au motif du peptide sélecteur dans le premier composant ;
(e) l'immobilisation des molécules de liaison identifiées dans l'étape (d) sur une matrice de telle sorte que les différents types de molécules de liaison soient immobilisés au niveau d'emplacements définis et discrets ;
(f) la fourniture d'un échantillon hétérogène de peptides, ou de fragments protéiques ou peptidiques, lequel échantillon comprend des peptides, ou des fragments protéiques ou peptidiques ayant chacun un motif qui est lié par une molécule de liaison immobilisée à l'étape (e) ;
(g) la séparation de l'échantillon hétérogène de peptides, ou de fragments protéiques ou peptidiques, en classes hétérogènes par liaison des membres de chaque classe aux molécules de liaison immobilisées à l'étape (e) ; et
(h) la caractérisation des peptides, ou des fragments protéiques ou peptidiques dans chaque classe par détermination de la masse des peptides, ou des fragments protéiques ou peptidiques dans les classes hétérogènes, et par détermination de l'abondance des peptides, ou des fragments protéiques ou peptidiques de masse différente dans les classes hétérogènes.
